# FASCICULE DE BREVET EUROPEEN

(11) **EP 0 721 314 B1**
(45) Date de publication et mention de la délivrance du brevet: **31.05.2000**
(21) Numéro de dépôt: 95925034.1
(22) Date de dépôt: 11.07.1995
(51) Int. Cl.: A61B 17/14

(54) **APPAREIL DE RESECTION DES CONDYLES DU GENOU POUR LA MISE EN PLACE D'UNE PROTHESE**
VORRICHTUNG ZUR RESEKTION VON KONDYLEN EINES KNIES ZUM EINZETZEN EINER KNIEPROTHESE
APPARATUS FOR RESECTIONING KNEE CONDYLES, ENABLING A PROSTHESIS TO BE FITTED

(30) Priorité: 12.07.1994 FR 9408670
(43) Date de publication de la demande: 17.07.1996
(73) Titulaire: BIOMICRON, 92110 Clichy (FR); ORTHO DIFFUSION & RECHERCHE, 14000 Caen (FR)
(72) Inventeur: DUVILLIER, Eric, F-75016 Paris (FR); GINESTON, Jean-Marie, F-92200 Neuilly (FR); DE ROALDES, Olivier, F-91310 Longpont-sur-Orge (FR)
(74) Mandataire: Benech, Frédéric
(86) Numéro de dépôt international: FR9500923
(87) Numéro de publication internationale: WO9601588

(56) Documents cités:
- EP-A- 0 538 153
- EP-A- 0 555 003
- FR-A- 2 664 157
- FR-A- 2 679 766
- FR-A- 2 681 779

## Description

La présente invention concerne un appareil de résection des condyles du genou pour la mise en place d'une prothèse, comprenant un dispositif de coupe fémorale du type comportant un élément support muni d'une embase inférieure propre à coopérer en appui avec les condyles fémoraux, un guide de lames de coupes fémorales, dites coupe antérieure, coupe en chanfrein antérieur, coupe en chanfrein postérieur, coupe postérieure et coupe distale, le guide étant fixé à l'élément support et comprenant au moins un bloc réglable en hauteur par rapport audit élément support, une tige intramédullaire fémorale d'orientation et de centrage du dispositif, et des moyens palpeurs de la partie corticale antérieure du fémur, solidaires du guide.

Elle trouve une application particulièrement importante, bien que non exclusive, dans le domaine de l'arthroplastie du genou, qui a pour objet la restauration de la fonction articulaire du genou dont les surfaces condyliennes ont été progressivement dégradées du fait de l'arthrose.

On sait que l'arthroplastie ne donne de bons résultats que si les prothèses sont mises en place avec précision, ce qui nécessite un appareil ancillaire de préparation permettant, d'une part, une orientation exacte des guides de coupe pour reproduire l'alignement mécanique du membre inférieur traité, et d'autre part, un équilibrage des coupes des surfaces articulaires tibiales et fémorales pour procurer une bonne stabilité sur toute la partie de l'articulation du genou.

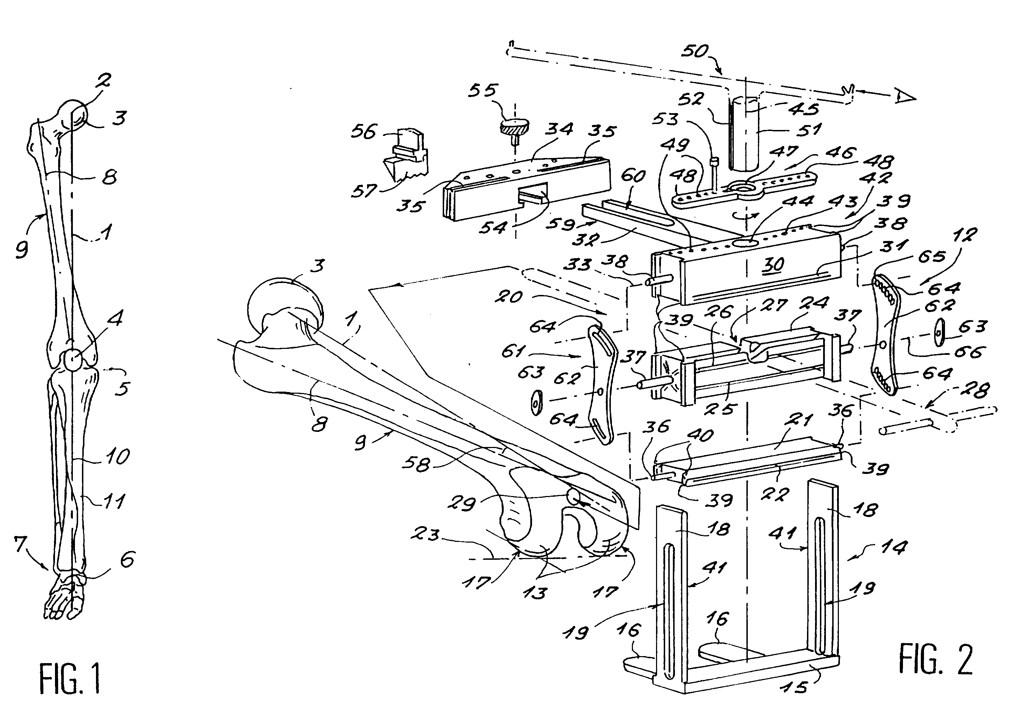

Pour ce faire, il est nécessaire que la prothèse respecte précisément l'axe mécanique 1 du membre inférieur traité (voir figure 1), axe qui passe par le centre 2 de la tête fémorale 3, par le centre 4 de l'articulation 5 du genou et par le centre 6 de l'articulation 7 de la cheville.

Or l'axe mécanique 1 forme avec l'axe anatomique 8 du fémur 9 un angle compris entre 3° et 9°, selon les individus, et se superpose par contre sensiblement avec l'axe anatomique 10 du tibia 11, sauf en cas de déformation.

La bonne orientation de l'appareil de résection, et sa mise en place de façon simple et fiable en fonction des différents axes, est donc primordiale pour le praticien.

On connaît déjà de nombreux appareils ancillaires de découpes fémorales et/ou tibiales.

Le document FR A 2.679.766 décrit par exemple un dispositif de coupe fémorale permettant un positionnement correct des implants prothétiques dans les plans frontal et sagittal.

Un tel dispositif présente cependant des inconvénients.

Il nécessite en effet plusieurs guides de lames de coupe de dimensions différentes pour s'adapter aux différentes tailles d'articulation. Il en résulte des risques d'erreurs lors du positionnement du guide, présélectionné le plus souvent par tâtonnement. Le temps de mise en place du guide est par ailleurs long, le coût et l'encombrement de l'ensemble des pièces du dispositif étant de plus importants.

On connaît également (FR-A-2 664 157) un appareil de résection comprenant un guide de lames de coupe en deux blocs, à savoir un premier bloc solidaire de l'élément support pour les coupes en chanfrein et la coupe postérieure et un deuxième bloc réglable en hauteur par rapport au premier pour effectuer la coupe antérieure.

Un tel appareil présente ici encore des inconvénients liés à son manque d'adaptabilité aux dimensions des condyles du patient.

Le document FR 2.629.339 décrit quant à lui un matériel de pose pour élément tibial comprenant une tige externe de guidage solidaire du guide de coupe tibiale et agencée pour être fixée temporairement sur une partie du tibia.

Un tel matériel présente également des inconvénients. Il est en effet peu fiable car difficile à régler et susceptible d'entraîner une coupe qui n'est pas parfaitement orthogonale à l'axe anatomique du tibia.

On connaît également des appareils de pose pour élément tibial comprenant une tige de positionnement intramédullaire.

De tels appareils ne sont pas non plus entièrement satisfaisants car ils ne permettent pas de modifier l'alignement du guide de coupe, par exemple pour rattraper une erreur initiale de centrage intramédullaire de la tige ou une déformation.

Pour tenter de remédier à ces inconvénients, le document FR-A-2 681 779 propose un appareil ancillaire comprenant une tige intramédullaire et une tige de visée externe permettant au praticien de réaliser deux types de visées.

Un tel appareil ne permet cependant pas au guide de coupe de s'adapter parfaitement au condyle de genou à couper.

La présente invention vise à fournir un appareil de résection des condyles du genou pour la mise en place d'une prothèse, répondant mieux que ceux antérieurement connus aux exigences de la pratique, notamment en ce qu'elle permet de n'utiliser qu'un seul guide de lames de coupe fémorales, et ceci quelque soit la taille des fémurs à traiter, en ce qu'elle utilise dans un mode de réalisation particulier un dispositif de coupe tibial qui s'articule dans le plan frontal et sagittal, et en ce qu'elle est simple et rapide à mettre en oeuvre par le praticien tout en garantissant un positionnement correct et fiable des guides de coupe.

L'invention permet, de plus, de choisir comme repère constant pour les coupes fémorales, soit le plan bi-condylien, soit le plan de la partie corticale antérieure du fémur, ce qui permet ainsi d'éviter les déséquilibres ligamentaires grâce à une plus grande adaptabilité au type de fémur rencontré.

Dans ce but, l'invention propose notamment un appareil de résection des condyles du genou pour la mise en place d'une prothèse, comprenant un dispositif de coupes fémorales du type ci-dessus défini,
caractérisé en ce que le guide de lames de coupe comporte au moins trois blocs dont au moins deux blocs sont mobiles par rapport audit élément support, lesdits blocs étant réglables en hauteur les uns par rapport aux autres, l'un des blocs comprenant des moyens de guidage d'au moins une coupe parmi les coupes antérieure, postérieure, en chanfrein antérieur et en chanfrein postérieur, et les autres blocs comprenant des moyens de guidage des coupes restantes parmi lesdites coupes antérieure, postérieure, en chanfrein antérieur et en chanfrein postérieur.

Dans des modes de réalisation avantageux, on a également recours à l'une et/ou à l'autre des dispositions suivantes :
- les trois blocs sont mobiles par rapport audit élément support et comprennent un bloc inférieur pour la coupe postérieure, un bloc central pour les coupes en chanfrein antérieur et postérieur, et un bloc supérieur pour la coupe antérieure ;
- le guide de lames de coupe comporte quatre blocs mobiles par rapport audit élément support, à savoir un bloc inférieur pour la coupe postérieure, deux blocs médians comprenant un premier bloc médian pour la coupe en chanfrein antérieur et un second bloc médian pour la coupe en chanfrein postérieur, et un bloc supérieur pour la coupe antérieure ;
- le dispositif de coupe fémorale comporte des moyens de réglage des écartements entre blocs de façon proportionnelle ;
- les moyens de réglage des écartements entre blocs de façon proportionnelle comprennent deux joues latérales disposées de part et d'autre des blocs, mobiles en rotation autour d'un axe central entre deux positions, à savoir une position où les blocs sont écartés au maximum les uns des autres, et une position où les blocs sont rapprochés les uns des autres, lesdites joues latérales étant munies de lumières en forme d'arc de cercle, propres à coopérer avec des pions solidaires des parois latérales extérieures des blocs mobiles entre eux ;
- le guide de lame de coupe comportant un bloc central pour les coupes en chanfrein, l'axe central est solidaire dudit bloc central et les joues latérales sont munies chacune de deux lumières en forme d'arc de cercle ou de courbe, symétriques par rapport audit axe central et propres à coopérer avec des pions respectivement solidaires des parois latérales des blocs inférieur et supérieur ;
- le guide de lame de coupe comportant deux blocs médians, l'axe central est solidaire d'un ou de deux éléments intercalaires disposés entre les deux blocs médians et les joues latérales sont munies chacune de quatre lumières en forme d'arc de cercle, symétriques deux à deux par rapport audit axe central, à savoir deux lumières extérieures propres à coopérer avec des pions respectivement solidaires des parois latérales des blocs inférieur et supérieur, et deux lumières intérieures propres à coopérer avec des pions respectivement solidaires des parois latérales des blocs médians ;
- les lumières desdites joues latérales sont crantées intérieurement, les crans étant propres à coopérer par encliquement avec lesdits pions ;
- le bord périphérique supérieur des joues est en courbe, cranté, de même forme courbe que les lumières, les moyens de réglage comprenant une pièce en forme de cornière munie de deux parties d'extrémité latérales dont les bords périphériques respectifs comportent une lèvre propre à coopérer avec les crans, ladite pièce étant basculante et mobile en rotation autour d'un axe solidaire du bloc supérieur, entre une position comprimée où les lèvres sont libérées des crans et une position de rappel par ressort où les lèvres sont encliquées dans lesdits crans ;
- l'embase inférieure de l'élément support comprend au moins une plaque amovible propre à coopérer en appui avec les condyles fémoraux ;
- le bloc inférieur est réglable en hauteur par rapport à l'embase inférieure sur une distance inférieure ou égale à une valeur déterminée ;
- l'élément support comporte deux paires de colonnes de guidage des blocs reliées entre elles par des pièces d'extrémité et situées de part et d'autre de l'appareil, lesdits blocs comprenant des alésages de passage desdites colonnes pour permettre le coulissement à frottement doux desdits blocs le long desdites colonnes entre les deux dites positions ;
- les moyens palpeurs de la partie corticale antérieure du fémur sont déplaçables latéralement par rapport à l'élément support ;
- le dispositif de coupe fémorale comporte des moyens de réglage de l'angle formé entre l'axe perpendiculaire au guide de lames de coupe et la tige intramédullaire ;
- les moyens de réglage de l'angle sont solidaires de la tige intramédullaire fémorale ;
- les moyens de réglage de l'angle sont solidaires du bloc supérieur ;
- le guide de lames de coupe comporte un bloc de coupe distale solidaire du bloc de coupe antérieure et muni d'au moins deux fentes de guidage de lames de coupe distale ;
- l'appareil comporte un dispositif de coupe tibiale muni d'une pièce de centrage apte à coopérer avec une tige intramédullaire tibiale, ledit dispositif de coupe tibiale comprenant une tige externe au tibia dont une extrémité est solidaire de ladite pièce et comporte un bloc de coupe muni d'une face courbe de contact avec le tibia, et dont l'autre extrémité est agencée pour être fixée temporairement sur une partie du tibia ;
- la tige externe au tibia solidaire de la pièce de centrage est fixée à ladite pièce via un élément intermédiaire agencé pour permettre un réglage de la position du bloc de coupe tibiale dans le plan frontal et dans le plan sagittal, par pivotement autour d'un centre coïncidant avec le centre de gravité ou sensiblement avec le centre de gravité dudit guide de coupe tibiale ;
- l'élément intermédiaire comprend une pièce ajourée en forme de portion de tube cylindrique définissant une lumière dans laquelle peut se déplacer l'extrémité de la tige externe, ladite extrémité étant munie de deux cales hémisphériques situées de part et d'autre de ladite pièce, lesdites cales étant propres à coopérer à frottement et à être maintenues en pression contre les surfaces cylindriques interne et externe de ladite pièce par des moyens de serrage, le centre des cales hémisphériques et l'axe du cylindre passant par le centre ou sensiblement le centre de gravité ou géométrique dudit bloc de coupe.

L'invention sera mieux comprise à la lecture de la description qui suit, de modes de réalisation donnés à titre d'exemple non limitatif.

La description se réfère aux dessins qui l'accompagnent dans lesquels :
- La figure 1, d'ores et déjà décrite, est une vue en élévation du squelette d'un membre inférieur humain.
- La figure 2 est une vue en perspective, démontée, d'un mode de réalisation du dispositif de coupe fémorale appartenant à un appareil selon l'invention, agencé pour coopérer avec un fémur en position fléchie.

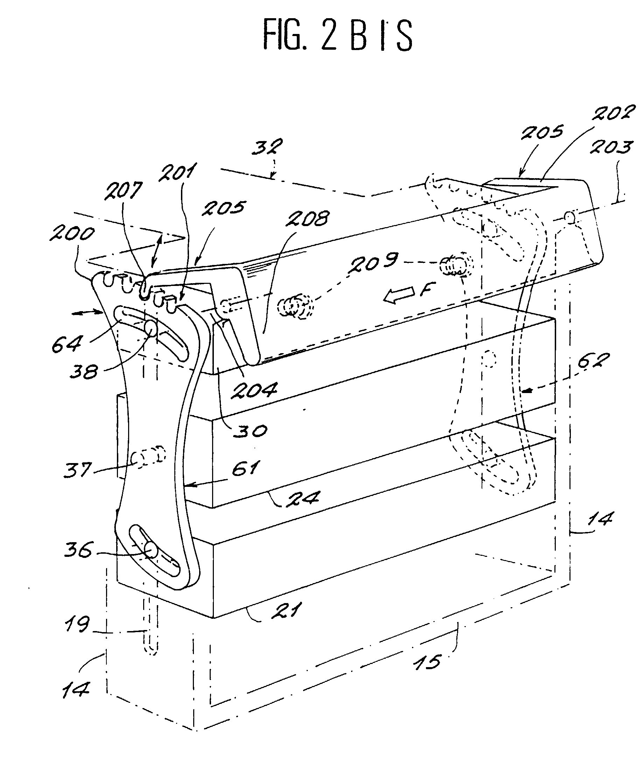

- La figure 2bis est une vue schématique, en perspective, d'une variante de réalisation des moyens de réglage de la distance entre blocs, selon l'invention.

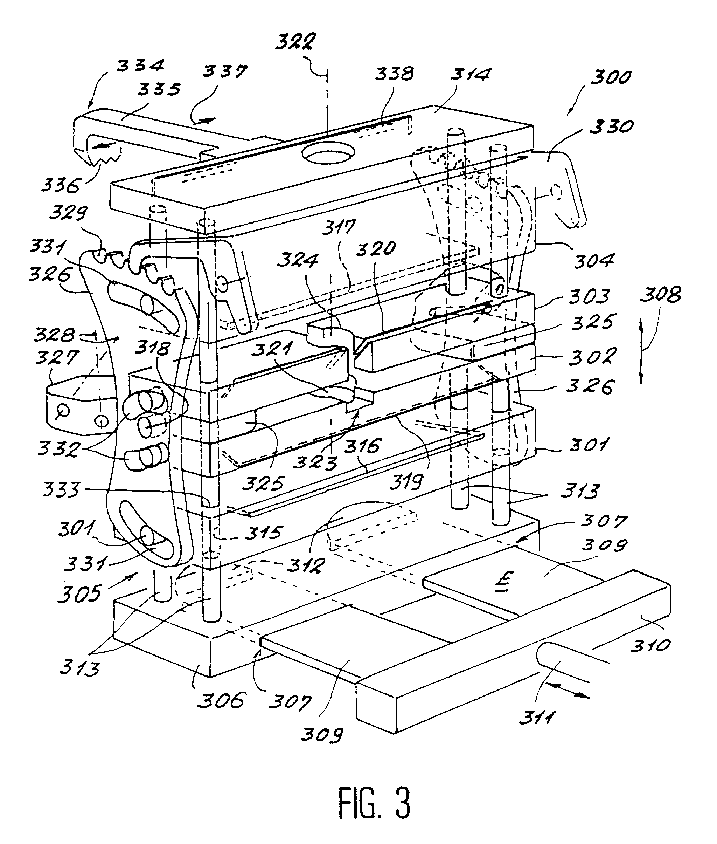

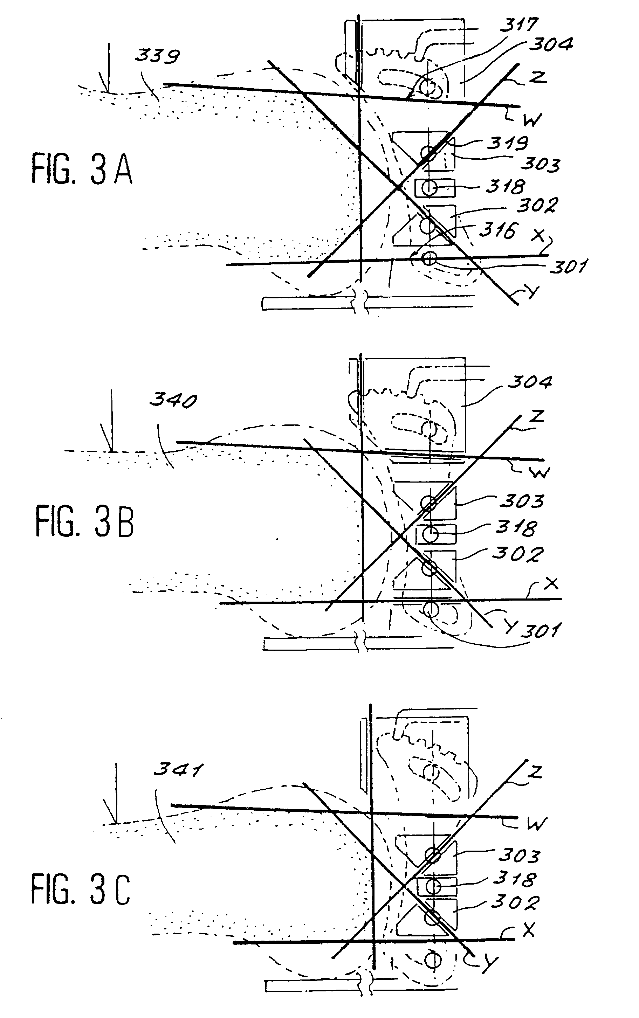

- La figure 3 est une vue schématique en perspective d'un autre mode de réalisation du dispositif selon l'invention.
- Les figures 3A, 3B et 3C sont des vues schématiques en coupe, montrant le fonctionnement et les coupes réalisées par le dispositif de la figure 3, avec des fémurs de tailles différentes.

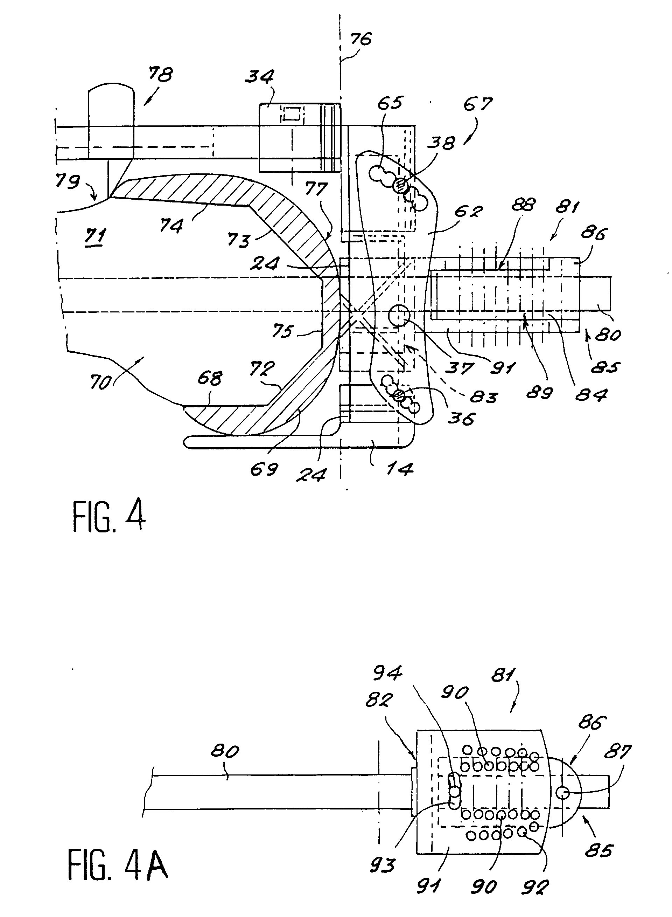

- La figure 4 est une vue latérale d'un autre mode de réalisation du dispositif de coupe fémorale selon l'invention.
- La figure 4A est une vue de dessus de la partie du dispositif de la figure 4 permettant le réglage en rotation du guide de coupe fémorale, par rapport à l'axe anatomique.

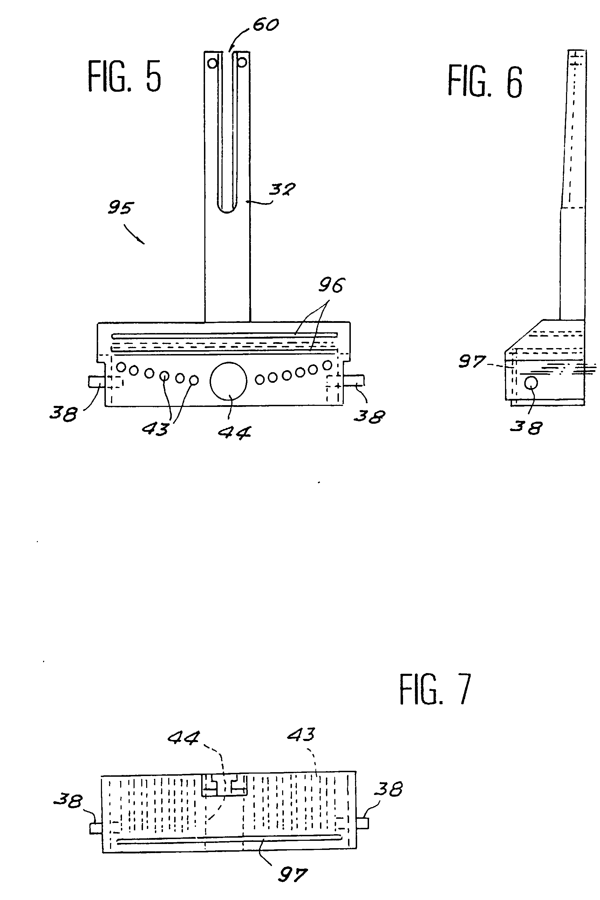

- Les figures 5 à 7 sont des vues respectivement de dessus, latérale et de face du bloc supérieur du guide de lames de coupes fémorales selon un autre mode de réalisation de l'invention, permettant le réglage en rotation du guide de coupe fémorale pour rattraper le différentiel d'angle entre axe anatomique et axe mécanique.

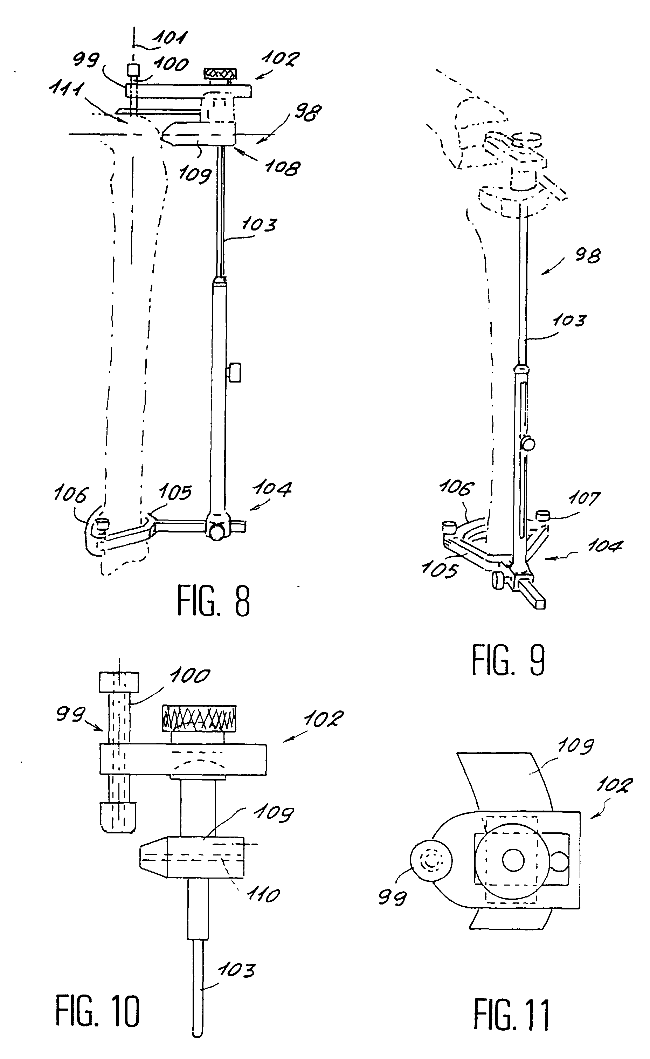

- La figure 8 est une vue latérale du dispositif de coupe tibiale selon un premier mode de réalisation de l'invention.
- La figure 9 est une vue schématique en perspective du dispositif de la figure 8.
- Les figures 10 et 11 sont des vues latérale et de dessus de la partie supérieure du dispositif des figures 8 et 9.

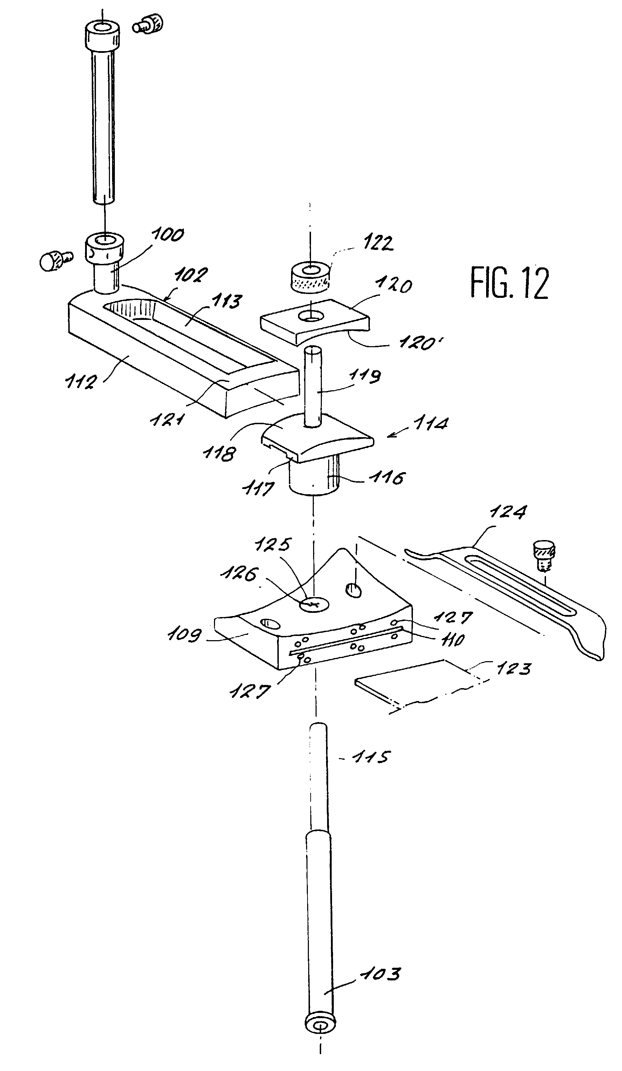

- La figure 12 est une vue en perspective, démontée, de la partie supérieure d'un mode de réalisation du dispositif de coupe tibiale similaire à celui décrit en référence aux figures 8 à 11.

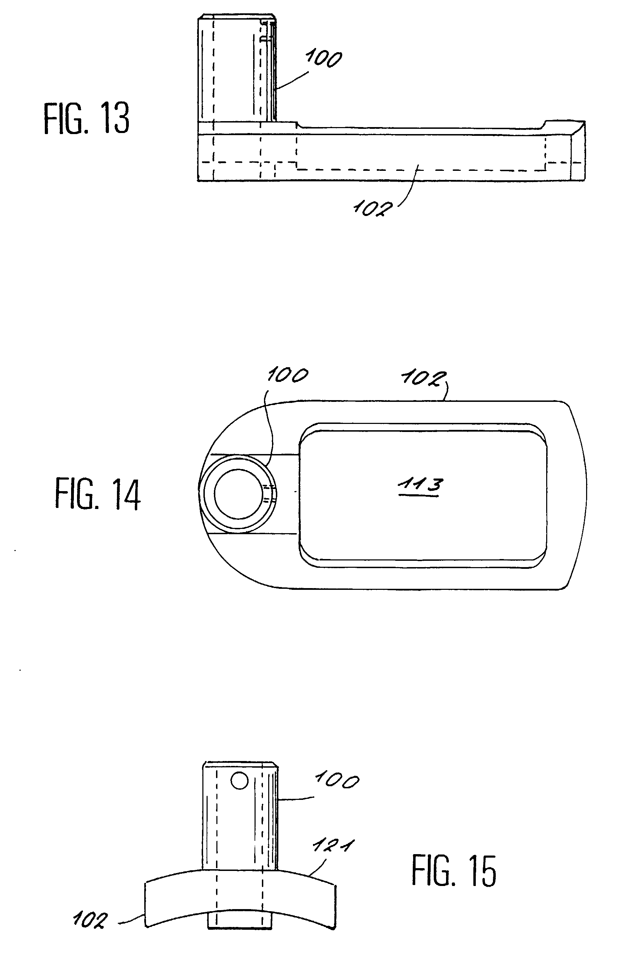

- Les figures 13, 14 et 15 sont des vues respectivement latérale, de dessus et de face, de la pièce ajourée du dispositif des figures 10 et 11.

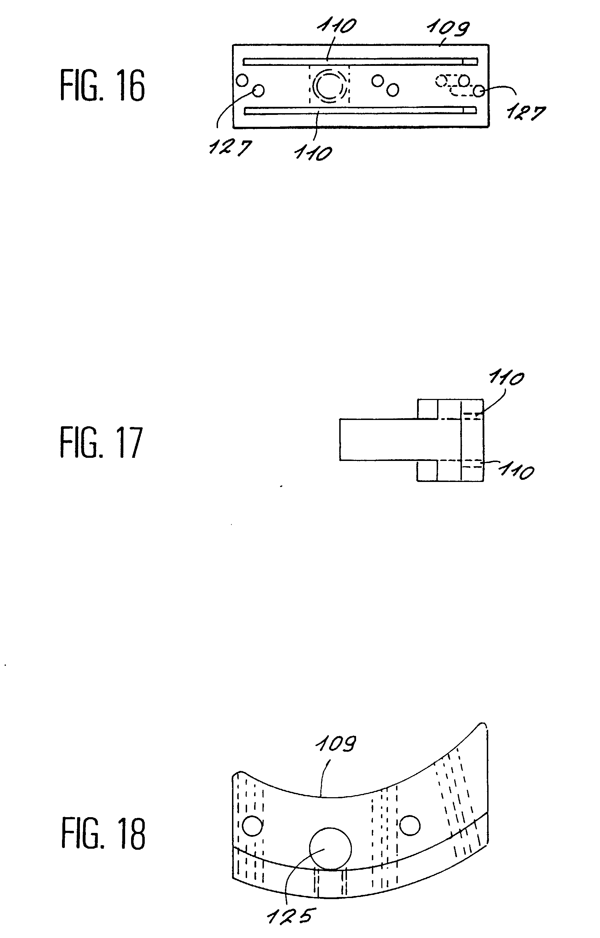

- Les figures 16, 17 et 18 sont des vues respectivement de face, latérale et de dessus du guide de coupe tibiale des figures 10 et 11.

Selon l'invention, l'appareil de résection des condyles du genou pour la mise en place d'une prothèse (non représentée) comprend un dispositif de coupe fémorale et avantageusement un dispositif de coupe tibiale.

Sur la figure 2, on a représenté en perspective et démonté, un dispositif 12 de coupe fémorale, en acier inoxydable, propre à couper les condyles 13 du fémur 9 de la figure 1, selon un premier mode de réalisation.

Le dispositif 12 comprend un élément support 14 comportant une embase inférieure en forme de plaque rectangulaire 15 munie sur un côté de deux languettes latérales 16, perpendiculaires à l'embase, plates, symétriques, chacune propre à coopérer en appui avec la surface inférieure 17 d'un condyle 13.

L'élément support 14 comprend deux montants latéraux verticaux 18, en forme de plaquette allongée, chacun muni d'une lumière longitudinale 19 sur l'essentiel de sa hauteur.

Le dispositif 12 comporte de plus un guide 20 de lames de coupe fémorale (non représentées) comprenant trois blocs en acier plein, mobiles dans le sens vertical, d'une part par rapport à l'élément support 14 et d'autre part l'un par rapport à l'autre.

Il s'agit tout d'abord d'un bloc inférieur 21 parallélépipédique, muni d'une fente 22 traversant le bloc transversalement de part en part, sensiblement parallèle aux languettes latérales 16 et agencé pour effectuer une coupe horizontale de la partie inférieure des condyles, dite coupe postérieure, parallèlement à la ligne condylienne postérieure 23 (le fémur étant classiquement placé en décubitus sur la table d'opération).

Il s'agit ensuite d'un bloc central 24 parallélépipédique, d'une seule pièce, de section transversale sensiblement carrée, comprenant deux fentes longitudinales 25 et 26, traversant le bloc transversalement de part en part du bas vers le haut pour l'une (25) et du haut vers le bas pour l'autre (26) en faisant l'une avec l'autre un angle de 90°, lesdites fentes étant agencées pour laisser passer des lames de coupes propres à réaliser les coupes des condyles dites coupe en chanfrein postérieur pour la fente 26 et coupe en chanfrein antérieur pour la fente 25.

Le bloc 24 comprend de plus un orifice conique 27 de passage transversal d'une tige intramédullaire 28 (en trait mixte sur la figure 2), en travers du bloc, d'orientation et de centrage du dispositif.

L'orifice conique 27 permet un déplacement angulaire de l'ensemble du dispositif autour de l'axe de la tige 28, pour rattraper l'angle existant entre les axes 1 et 8 du fémur.

La tige 28 est quant à elle introduite dans le canal médullaire du fémur centré autour de l'axe anatomique 8 de ce dernier, par un orifice 29 préalablement percé au centre de l'articulation.

Enfin le dispositif 12 comprend un bloc supérieur 30, parallélépipédique, muni d'une fente 31 sensiblement parallèle à la fente 22 du bloc 21, traversant transversalement le bloc 30 de part en part et agencé pour effectuer une coupe horizontale ou sensiblement horizontale, inclinée légèrement vers le haut, par exemple de 3°(cône morse) dite coupe antérieure, de la partie supérieure des condyles du fémur en décubitus.

Le bloc 30 est par ailleurs muni d'une tige latérale 32, perpendiculaire à l'axe longitudinal 33 du bloc 30 et centrée par rapport audit bloc.

La tige 32 supporte un quatrième bloc mobile 34, de coupe verticale des condyles dite coupe distale, perpendiculaire à l'axe mécanique 1 du fémur.

Le bloc 34 comprend deux fentes verticales symétriques 35, situées de part et d'autre du bloc, et traversant les extrémités dudit bloc de part en part sur une distance longitudinale correspondant à la largeur des deux extrémités condyliennes.

Les blocs 21, 24 et 30 comprennent par ailleurs sur chacune de leurs faces latérales transversales un téton axial cylindrique, respectivement les tétons 36, 37 et 38, propres à coopérer à glissement avec la lumière 19 du montant 18 en vis à vis, lumière qui sert donc de rail de guidage vertical de part et d'autre desdits blocs.

Des joues 39 en saillies latérales, prolongeant les faces verticales longitudinales de chaque bloc, situées de part et d'autre de ceux-ci, et dont les faces internes 40 constituent des surfaces de friction destinées à coagir avec les tranches latérales verticales 41 du montant 18 correspondant, complètent le guidage en empêchant le basculement des blocs vers l'avant ou l'arrière.

Les blocs 21, 24 et 30 sont de même largeur et de même longueur. Ils présentent ainsi une face vers les condyles du fémur dans un seul et même plan.

Compte tenu de l'angle existant entre l'axe anatomique 8 du fémur, qui coïncide avec celui de la tige 28 quant elle y est introduite, et l'axe mécanique 1, des moyens 42 de réglage angulaire de la position de l'ensemble du dispositif 12 par rapport à la tige 28 sont par ailleurs prévus.

Les moyens 42 comportent des alésages verticaux 43 de petits diamètres, percés dans le bloc 30. Les alésages 43 sont répartis en deux lignes divergentes à partir d'un alésage 44, cylindrique, central, de plus grand diamètre, vertical, allongé autour de l'axe 45 du dispositif.

Les lignes forment avec l'axe 38 du bloc 30, respectivement et de part et d'autre de l'alésage 44, un angle par exemple égal à 10°.

Les moyens 42 comprennent également une plaquette 46 munie d'une partie centrale comportant un orifice axial 47 et deux pattes d'extrémités 48, dans le prolongement l'une de l'autre, percées de part en part par de petits alésages cylindriques 49, de diamètres égaux à ceux des alésages 43, et répartis sur une ligne axiale de la plaquette.

La plaquette 46 est agencée pour reposer sur la face supérieure du bloc 30, l'orifice axial 47 étant alors centré par rapport à l'alésage 44 du bloc 30.

Le dispositif 12 comporte de plus un système 50 connu en lui-même, de visée du centre de la tête fémorale et propre à déterminer l'axe mécanique 1.

Le système 50 est solidaire en rotation de la plaquette 46, et comprend une tige 51 avec clavette 52, propre à coopérer avec un évidement latéral de l'orifice 47 en vis-à-vis (non représenté).

Une pinoche 53 agencée pour être introduite dans les petits alésages 43 et 49, en vis-à-vis, permet de positionner les blocs et notamment le bloc 34 de coupe distale, solidaire du bloc 30, de façon à ce que la coupe distale soit perpendiculaire à l'axe mécanique 1 matérialisé grâce au système de visée 50.

Le bloc 34 comprend par ailleurs, sur sa face inférieure, un évidement transversal, central 54, ledit évidement étant propre à coopérer à frottement avec la tige 32 le long de laquelle le bloc se déplace et à laquelle il peut être fixé par une molette de compression 55.

Un curseur 56 muni d'une lame palpeuse 57 de la partie corticale antérieure 58 du fémur, est par ailleurs prévu pour coulisser le long de l'extrémité 59 de la tige 32, dans une échancrure de guidage 60.

Autrement le curseur 56 est solidaire de la tige 32 sur les deux degrés de liberté restants.

Une telle disposition permet d'adapter la position du dispositif en fonction de la dimension des condyles, l'extrémité 59 présentant une légère pente dirigée vers le bloc 30.

Le dispositif 12 comprend par ailleurs des moyens 61 de réglage des écartements entre blocs de façon proportionnelle.

Les moyens 61 comprennent deux joues latérales 62 en forme de plaque munie d'une partie centrale de plus faible largeur que ses parties d'extrémité.

Les joues sont montées en rotation autour des tétons 37, de part et d'autre du bloc 24. Elles sont fixées longitudinalement par des écrous 63 de blocage latéral et comprennent respectivement sur leur partie d'extrémité des lumières 64 en forme d'arc de cercle, ou en courbe, munies de crans intérieurs 65 en même nombre, par exemple six, et disposés sur des droites concentriques avec les tétons 37.

Les lumières sont notamment disposées symétriquement par rapport à l'axe passant par les tétons 37.

Les crans 65 sont agencés pour coopérer par encliquement avec les deux pions 38 pour les lumières supérieures, et avec les deux pions 36 pour les lumières inférieures, lesdites lumières étant inclinées par rapport à l'horizontal pour que les blocs 21 et 24 s'écartent proportionnellement par rapport à l'écartement entre les blocs 24 et 30, par exemple dans un rapport de 1,3.

En faisant pivoter les joues en avant ou en arrière, les blocs se déplacent donc verticalement, guidés par des rails constitués par les montants 18 et les lumières 19, entre une position où les trois blocs sont écartés au maximum, et une position où ils sont les uns contre les autres.

La figure 2bis montre une variante des moyens de réglage selon l'invention.

Ici les joues latérales 62 comprennent un bord périphérique supérieur arrondi 200, muni de six crans 201 identiques régulièrement répartis, correspondant à des tailles de fémur différentes.

Une pièce basculante 202 est prévue. Elle est mobile en rotation autour d'un axe horizontal 203 parallèle au, et solidaire du, grand côté du bloc supérieur 30. Plus précisément, la pièce 202 est en forme de cornière dont l'aile supérieure est évidée sauf sur les côtés, formant de ce fait deux pattes latérales 205 s'étendant au delà du bloc 30, dans le sens de l'axe 203, ladite cornière s'étendant sur toute la longueur du bloc, de l'autre côté dudit bloc par rapport à la position du fémur, l'axe horizontal 203 étant solidaire de l'angle interne de ladite cornière qui est donc à cheval sur le coin supérieur 204 du bloc 30.

Les pattes latérales 205 s'étendant sensiblement parallèlement et à l'extérieur de chaque côté de la face supérieure 206 du bloc 30, sont chacune terminées à leur extrémité par une lèvre 207 dirigée vers le bas, propre à coopérer avec les crans 201, le bord périphérique 200 des joues 62 s'étendant en dessous de la face supérieure 206 du bloc 30, dans ce mode de réalisation.

L'autre aile 208 de la pièce 202 s'étend quant à elle vers le bas.

Elle est agencée pour être repoussée vers l'extérieur du bloc 30 par des moyens pousseurs, par exemple deux ressorts à boudin 209, symétriques par rapport au centre de l'aile, ressort dont un côté est solidaire et s'appuie sur le bloc 30 et l'autre sur la face interne de l'aile 208.

L'angle de la cornière est compris entre 93 et 100°, par exemple 95°. Lorsqu'en maintenant les blocs d'une main, l'opérateur appuie avec les doigts sur l'aile 208, contrecarrant l'action des ressorts, les lèvres 107 sont écartées des crans et les joues peuvent pivoter librement, écartant et rapprochant les blocs entre eux.

Une fois la position d'écartement optimum obtenue, correspondant ou correspondant sensiblement à la taille des condyles du patient, la pression sur l'aile 208 est relâchée.

Grâce au ressort, l'aile 208 s'écarte du bloc 30 en pivotant autour de l'axe 203, abaissant les pattes 205 et les lèvres 207 qui viennent ainsi coopérer avec les crans en vis à vis.

Les figures 3, 3A, 3B et 3C montrent un dispositif 300 de coupe fémorale comprenant quatre blocs en acier inoxydable 301, 302, 303 et 304, mobiles par rapport à l'élément support 305, à savoir un bloc inférieur 301, pour la coupe inférieure X, deux blocs médians 302 et 303 respectivement pour la coupe en chanfrein antérieure Y et pour la coupe en chanfrein postérieure Z, et un bloc supérieur 304 pour la coupe antérieure W.

L'élément support 305 comporte un socle 306 parallélépipédique comprenant deux fentes rectangulaires 307 transversales, parallèles aux grandes faces du socle et le traversant de part en part perpendiculairement à la direction 308 du mouvement des blocs entre eux.

L'élément support comporte deux plaques 309 amovibles par rapport au socle, propres à coopérer à frottement doux avec les fentes 307 et reliées entre elles par une base horizontale 310 et actionnable manuellement par le praticien via une tige 311 entre une position de désengagement total des plaques, et une position de pénétration maximale des plaques où la base 310 vient en butée avec le socle de sorte que les extrémités 312 desdites plaques ressortent entièrement de l'autre côté du socle pour constituer les éléments d'embase inférieur propres à coopérer en appui avec les condyles fémoraux.

L'élément support 305 comporte par ailleurs deux paires identiques de colonnes 313 par exemple cylindriques, disposées transversalement par rapport au bloc 306 de guidage très précis et de maintien latéral des blocs.

Les colonnes sont fixées d'un coté sur le socle 306 et de l'autre coté à une pièce supérieure 314.

Chaque bloc est percé de part en part et de part et d'autre de deux alésages cylindriques 315, de passage des colonnes 313, pour permettre leur coulissement à frottement doux le long desdites colonnes.

Plus précisément, les blocs inférieur 301 et supérieur 304 sont par exemple constitués chacun par une plaque épaisse parallélépipédique munie respectivement d'une fente 316 (317) traversant les blocs transversalement, sensiblement parallèles aux plaques 309 et agencées pour effectuer respectivement une coupe horizontale X de la partie inférieure des condyles, et une coupe légèrement inclinée par rapport à l'horizontale W, dite coupe antérieure, de la partie supérieure des condyles du fémur.

Les blocs médians 302 et 303 sont par exemple de section transversale sensiblement en forme de trapèze rectangle dont la base est située vers l'extérieur du dispositif par rapport à l'os et est parallèle à la direction 308, et dont le coté dirigé vers l'os forme un angle de 45° par rapport à l'horizontal, symétriquement par rapport à l'axe central 318 parallèle aux blocs.

Les blocs médians 302 et 303 comprennent chacun une fente longitudinale, respectivement 319 et 320, traversant les blocs transversalement de part en part du bas vers le haut pour le bloc 302 et du haut vers le bas pour le bloc 303 en faisant l'une avec l'autre un angle de 90° (deux fois 45°), et propres à réaliser les coupes des condyles dites coupes en chanfrein antérieur Y pour la fente 319 et chanfrein postérieur Z pour la fente 320.

Plus précisément, le bloc médian est par exemple monobloc et comprend un culot 321 de réception de la partie inférieure de l'axe de pivotement symbolisé en 322, et une encoche 323 qui va permettre le guidage et le passage de la tige intramédullaire de positionnement (non représentée).

Le bloc médian supérieur 303 est quant à lui, par exemple, constitué de deux petits blocs identiques, symétriques, muni chacun d'une face 324 de guidage avec évidement semi-cylindrique, de l'axe 322. Il peut également être monobloc.

L'élément support comprend par ailleurs deux pièces intercalaires 325 situées entre les deux blocs médians et non solidaires desdits blocs, munies respectivement sur leur face latérale externe d'un pion d'extrémité constituant l'axe 318 et formant les axes respectifs des joues latérales 326.

Les pièces intercalaires 325 comprennent des parties d'extrémité 327 s'étendant latéralement au delà du dispositif et agencés pour permettre la fixation du dispositif sur le fémur par des clous 328.

Les joues 326 sont du type de celles décrites en référence à la figure 2 bis, c'est à dire munies de crans 329 sur les bords périphériques supérieurs et agencés pour coopérer avec la pièce basculante 330 par exemple identique à la pièce basculante 202 de ladite figure 2 bis.

Plus précisément chaque joue latérale 326 est munie de quatre lumières en forme d'arc de courbe, symétriques deux à deux par rapport à l'axe central 318, à savoir deux lumières extérieures 331 chacune propre à coopérer avec un pion respectivement solidaire de la paroi latérale externe des blocs inférieur et supérieur, et deux lumières intérieures 332 chacune propre à coopérer avec un pion respectivement solidaire de la paroi latérale des blocs médians inférieur et supérieur, les arcs de courbes des lumières 332 étant de sens opposés aux arcs de courbes 331.

Dans le mode de réalisation de la figure 3, le bloc inférieur 301 est mobile et réglable en hauteur par rapport au socle 306 sur une distance limitée, ne pouvant dépasser par exemple 2 cm, grâce à une butée mécanique 333 constituée par une excroissance annulaire sur l'une des colonnes 313.

Avantageusement les moyens palpeurs 334 comprennent une tige 335, à laquelle est fixée rigidement une lame palpeuse 336, la tige étant montée mobile dans le sens latéral 337 sur un rail transversal 338 interne à la pièce 314 rendant ainsi les moyens palpeurs réglables latéralement pour s'adapter au fémur concerné.

On va maintenant décrire le fonctionnement du dispositif selon le mode de réalisation de la figure 3 en référence aux figures 3A, 3B et 3C.

Chaque figure correspond à un fémur de taille différente, du plus grand vers le plus petit.

On voit ainsi que grâce à un mode de réalisation de l'invention, du fait de l'écartement proportionnel entre blocs, réalisé automatiquement entre l'embase inférieure dont la position dépend des condyles inférieurs, et le bloc supérieur dont la position est réglée grâce aux moyens palpeurs, les découpes X, Y, Z et W sont effectuées sur des longueurs d'os non fixées, mais au contraire adaptées aux fémurs concernés de façon souple et optimisée.

Ainsi pour un gros fémur 339, les distances entre blocs 303 et 302 d'une part, et entre blocs 301 et 304 d'autre part sont agrandies, puis réduite (figure 3B, puis figure 3C) au fur et à mesure que les dimensions du fémur 340 et 341 diminuent, adaptant ainsi les longueurs de découpes horizontales ou sensiblement horizontales (X et W), ou à 45° (Y, Z) au fémur concerné.

La figure 4 est une vue schématique latérale d'un second mode de réalisation d'un dispositif 67 de coupe fémorale.

Pour simplifier, on reprendra dans les descriptions ci-après les mêmes numéros de référence que ceux de la figure 2, pour désigner les mêmes éléments.

Le dispositif 67 comprend un élément support 14, quatre blocs de coupe, à savoir un bloc inférieur 21 de coupe postérieure 68 des parties condyliennes 69 de l'extrémité 70 du fémur 71, un bloc central 24 de coupe en chanfrein postérieur 72 et en chanfrein antérieur 73, un bloc supérieur 30 de coupe antérieure 74, et un bloc 34 de coupe distale 75, parallèle à la face commune 76 des blocs 21, 24 et 30, qui vient en appui sur la face externe 77 des parties condyliennes.

Des moyens palpeurs 78 de la partie corticale 79 antérieure du fémur sont par ailleurs prévus pour l'adaptation du dispositif aux dimensions de l'extrémité 70 du fémur du patient traité.

Le dispositif 67 comprend de plus une tige fémorale intramédullaire 80 munie à son extrémité d'un bloc 81 de réglage de l'angle existant entre ladite tige 80 d'une part et la face 82 du bloc d'autre part, face qui est agencée pour être appliquée sur, et coplanaire avec la face externe verticale 83 du bloc central 24.

Plus précisément (voir également figure 4A), le bloc 81 comprend une pièce 84 munie d'un alésage cylindrique interne longitudinal enfilé sur l'extrémité 85 de la tige à laquelle il est fixé par une extrémité 86 grâce à une clavette 87.

La pièce 84 comprend deux surfaces planes 88 et 89, supérieure et inférieure, du côté de son autre extrémité, respectivement percées de part en part, le long de, et parallèlement à, leur deux périphéries longitudinales, d'une ligne de trous verticaux 90, par exemple sept trous, de faible diamètre, par exemple trois millimètres.

Le bloc 81 comprend de plus une seconde pièce 91 en forme de mâchoire présentant une section transversale en U percée d'un alisage axial et central de passage de la tige 80, pièce dont la base comporte la face verticale 82 agencée pour être appliquée sur la face externe 83 du bloc 24, et dont les faces internes des branches du U coopèrent à friction avec les faces planes 88 et 89.

La pièce 91 est telle qu'elle permet de faire pivoter la face 82 par rapport à l'axe de la tige 80 de part et d'autre de la position perpendiculaire.

Pour ce faire elle comprend sur chaque bord périphérique longitudinal de la mâchoire en U, une série de trous 92, disposés à la suite les uns des autres le long d'un arc de cercle de grand diamètre ou d'une droite faiblement inclinée (2 à 3°) par rapport à l'axe de l'alésage de la pièce en U, à l'extérieur des trous 90 lorsque les pièces 84 et 91 sont dans le même axe.

Une pinoche (non représentée) introduite dans un trou 90 et un trou 92 en vis à vis, après pivotement des pièces 84 et 91 l'une par rapport à l'autre, pour rendre deux trous 90 et 92 co-axiaux, permet ainsi un positionnement fixe de la face 82 par rapport à la tige 80 avec un angle déterminé.

Une lumière en arc de cercle 93 située de façon centrée dans la branche supérieure de la pièce 91, et coopérant avec un pion 94 solidaire de la pièce 84 et de la tige 80, assure le guidage de la pièce 91 en rotation autour de l'axe de la clavette 87.

On a représenté sur les figures 5, 6 et 7 un autre mode de réalisation du bloc supérieur 95 du dispositif selon l'invention.

Dans ce cas, le bloc supérieur est solidaire, d'une seule pièce, avec le bloc de coupe distale. Il comprend donc d'une part deux fentes verticales 96 de coupe distale, et d'autre part une fente 97 sensiblement horizontale de coupe antérieure.

Une tige 32, et des orifices 43 et 44 comme décrit ci-avant sont par ailleurs prévus.

Les figures 8, 9, 10 et 11 montrent un mode de réalisation du dispositif 98 de coupe tibiale appartenant à l'appareil selon l'invention.

L'appareil 98 comprend une pièce 99 de centrage constituée par un manchon cylindrique 100 de passage d'une tige intramédullaire tibiale 101, introduite de facon connue en elle-même, le manchon étant fixé à un élément intermédiaire 102 de support d'une tige externe 103.

La tige 103 comprend, en partie inférieure, des moyens 104 réglables en hauteur, de fixation sur la cheville, ou à proximité, par l'intermédiaire d'une pièce en forme de V 105 fermée par une pièce courbe 106 articulée à une extrémité 107 et propre à venir enserrer le tibia en position refermée.

La tige 103 est solidaire en partie supérieure 108 d'un bloc 109 de coupe tibiale muni d'une fente transversale 110, de coupe des condyles 111 de l'os du tibia.

En référence aux figures 12 à 18 et plus précisément l'élément intermédiaire 102 comprend une pièce 112 ajourée en forme de portion de cylindre définissant une lumière 113 dans laquelle peut se déplacer un ensemble 114 solidaire de l'extrémité 115 de la tige externe 103.

Le manchon d'extrémité 100 de passage de la tige intramédullaire est fixé à une extrémité de la pièce 112.

L'ensemble 114 comporte, fixés et enfilés sur l'extrémité 115 de la tige et successivement du bas vers le haut :
. le guide 109 de lame de coupe ;
. une première cale constituée par un cylindre vertical 116 coiffée d'un chapeau 117 dont la face supérieure 118 est sphérique ou sensiblement sphérique, de plus grandes dimensions transversales que la largeur de la lumière 113, sur laquelle est fixée une tige 119 alignée axialement avec le cylindre 116 perpendiculairement à la face 118 ;
. la pièce ajourée 112, placée de façon mobile autour de cette tige ;
. une deuxième cale 120 comprenant une surface inférieure 120' sphérique ou sensiblement sphérique, de plus grandes dimensions transversales que la largeur de la lumière 113, agencée pour coopérer avec la face supérieure 121 du cylindre, (dont la face inférieure 121' est propre à coopérer avec la face supérieure 118 de la première cale) et
. une vis moletée 122 permettant de serrer les pièces 117 et 120 sur la pièce ajourée, en donnant une inclinaison déterminée dans le plan frontal et sagittal du guide de coupe 109, le centre géométrique 126 de ce dernier étant le centre ou sensiblement le centre des rayons des parois 118, 120', 121 et 121'.

Le guide de coupe 109 comprend par ailleurs une fente de passage 110 de la lame de coupe 123, et un palpeur 124, amovible, de la surface supérieure du condyle à couper, palpeur connu en lui-même.

Un orifice central 125 de passage de l'extrémité 115 de la tige 103 est par ailleurs prévu au niveau du centre géométrique 126 du guide.

Des orifices 127 de fixation du guide de coupe sur le tibia sont également prévus de façon connue.

On va maintenant décrire la mise en place et le fonctionnement d'un appareil de résection des condyles du genou selon l'invention, en faisant plus particulièrement référence aux figures 2 et 12.

Après ouverture du genou par le chirurgien, le genou étant placé en flexion, on met en place la tige intramédullaire 28, le point d'introduction se situant au sommet de l'échancrure intercondylienne, dans l'alignement du fémur, l'os sous-chondral ayant été percé au préalable avec une mèche, par exemple de diamètre 6 mm.

Puis on met en place l'élément support 14 en appui sur les condyles fémoraux 13, en enfilant sur la tige 28 le guide 12 de lames de coupes fémorales fixé à l'élément support 14 avec lequel il est réglable en hauteur, jusqu'à ce que le plan des faces coplanaires des blocs vienne en contact avec l'extrémité condylienne.

On oriente alors le guide de lame dans le plan sagittal en l'ajustant pour tenir compte de l'angle existant entre l'axe mécanique 1 du membre inférieur et l'axe anatomique 8 du fémur, grâce au système de réglage 42, la pinoche 43 étant introduite dans les trous 48 et 49 en vis à vis correspondant à l'angle observé par les moyens viseurs 50.

A l'aide des moyens palpeurs 56, 57 on positionne par ailleurs le bloc 30 de coupe antérieure à la place nécessaire tout en réglant les écartements entre les guides de lames de coupe comme décrit, en faisant pivoter les joues 62 autour de l'axe 66 de façon à écarter ou rapprocher les blocs 21 et 30 du bloc 24, les pions 38 et 36 étant librement déplaçables dans les lumières 64.

Puis on rapproche lesdites joues des blocs, les pions s'encliquant alors dans les crans 65 desdites lumières, manuellement ou automatiquement par un système de rappel à ressort (non représenté).

Les distances entre le trou de passage du pion 37, passant par l'axe 66, et les crans de deux lumières en vis à vis situés sur une même droite passant par ledit trou, sont dans un même rapport arithmétique, la distance entre les crans de la lumière supérieure et le trou étant par ailleurs décroissante du haut vers le bas dans des proportions allant par exemple de 1 à 0,7.

Compte tenu de la disposition des lumières, l'écartement entre le guide 21 de lames de coupe antérieur et le guide 24 de lames de coupe de chanfreins antérieur et postérieur d'une part, et entre ledit guide 24 de lames de coupe de chanfreins antérieur et postérieur et le guide 30 de lame de coupe postérieur d'autre part, est ainsi réglable de façon proportionnelle.

Une fois les guides parfaitement positionnés, on fixe l'élément support au fémur par des vis latérales connues en elles-mêmes, traversant des points de fixation (non représentés) solidaires du dispositif et on retire la tige intramédullaire.

On règle ensuite, dans le cas ou le bloc de coupe distale est mobile par rapport au bloc 30, la position transversale dudit bloc de coupe distale et on le fixe par vissage de la molette 55.

On réalise alors les coupes postérieure, en chanfrein postérieur, en chanfrein antérieur, antérieure et distale en une seule fois, sans modification des réglages ni évacuation des morceaux d'os entre deux coupes, ce qui constitue l'un des avantages de l'invention.

Avant de réaliser les coupes fémorales, ou après, on procède également à la préparation de l'os du tibia pour mise en place d'une prothèse tibiale propre à coopérer avec la prothèse fémorale destinée à être fixée sur la partie condylienne de l'os fémoral découpée avec le dispositif ci-dessus.

Pour ce faire, on introduit de plus la tige intramédullaire 101 tibiale dans le tibia et on règle le positionnement d'un guide 109 de coupe tibiale dans le plan horizontal et sagittal, via une tige 103 externe de rattrapage.

Plus précisément, on fore un trou centré médiolatéralement, on introduit la tige 101 dans le canal centromédullaire, on place le palpeur 124 en l'appuyant sur la partie médiane du plateau sein du condyle du tibia, on met en place la pièce 102, puis on met en place la tige extramédullaire en la fixant à distance à l'aide de la pince malléolaire 104 de sorte que la branche du V ou du Y soit alignée sur le deuxième métatarsien.

On vérifie alors l'alignement. Dans le plan frontal, la tige 103 doit être dans le prolongement du 2ème métatarsien en distal et en regard du bord interne de la tubérosité tibiale en proximal.

Dans le plan sagittal, la tige est parallèle à l'axe mécanique du tibia et non à la crête tibiale.

Le niveau de coupe est alors déterminé grâce au palpeur 124 comprenant une plaquette à bord dentelé, le guide de coupe étant déplacé en rotation autour de son centre géométrique, par dévissage de la molette 122, et orientation du guide par pivotement des surfaces 120, 120', 118 et 121 l'une contre l'autre.

On fixe alors l'élément support au fémur par des vis latérales de façon connue en elles-mêmes et on retire la tige intramédullaire. Puis on effectue la coupe tibiale.

L'ensemble de l'appareil est alors retiré, les fémur et tibia du patient étant prêts pour la mise en place des prothèses.

La présente invention ne se limite pas aux modes de réalisation plus particulièrement décrits ici. Elle en embrasse au contraire toutes les variantes et notamment celles où :
- la pièce support 14 comporte deux montants latéraux par exemple cylindriques, agencés pour coopérer avec des orifices correspondants percés de chaque côté dans les blocs 21, 24 et 30, pour effectuer le guidage et le maintien en place desdits blocs.

## Revendications

1. Appareil de résection des condyles du genou pour la mise en place d'une prothèse, comprenant un dispositif (12) de coupes fémorales comportant :
. un élément support (14) muni d'une embase inférieure (15, 16) propre à coopérer en appui avec les condyles fémoraux (17),
. un guide (21, 24, 30) de lames de coupes fémorales dites coupe antérieure, coupe en chanfrein antérieur, coupe en chanfrein postérieur, coupe postérieure et coupe distale, ledit guide étant fixé et comprenant au moins un bloc réglable en hauteur par rapport à l'élément support,
. une tige (28, 80) intramédullaire fémorale d'orientation et de centrage dudit dispositif et
. des moyens palpeurs (56, 57, 78) de la partie corticale (58,79) antérieure du fémur, solidaires dudit guide, caractérisé
en ce que le guide de lames de coupe comporte au moins trois blocs dont au moins deux blocs (21, 24, 30) sont mobiles par rapport audit élément support, lesdits blocs étant réglables en hauteur les uns par rapport aux autres, l'un des blocs comprenant des moyens de guidage d'au moins une coupe parmi les coupes antérieure, postérieure, en chanfrein antérieur et en chanfrein postérieur, et les autres blocs comprenant des moyens de guidage des coupes restantes parmi lesdites coupes antérieure, postérieure, en chanfrein antérieur et en chanfrein postérieur.

2. Appareil selon la revendication 1, caractérisé en ce que les trois blocs (21, 24, 30) sont mobiles par rapport audit élément support et comprennent un bloc inférieur (21) pour la coupe postérieure, un bloc central (24) pour les coupes en chanfrein antérieur et postérieur, et un bloc supérieur (30) pour la coupe antérieure.

3. Appareil selon la revendication 1, caractérisé en ce que le guide de lames de coupe comporte quatre blocs mobiles par rapport audit élément support, à savoir un bloc inférieur pour la coupe postérieure, deux blocs médians comprenant un premier bloc pour la coupe en chanfrein antérieur et un second bloc pour la coupe en chanfrein postérieur, et un bloc supérieur pour la coupe antérieure.

4. Appareil selon l'une quelconque des revendications précédentes, caractérisé en ce que le dispositif comporte des moyens (61) de réglage des écartements entre blocs de façon proportionnelle.

5. Appareil selon la revendication 4, caractérisé en ce que les moyens (61) de réglage des écartements entre blocs de facon proportionnelle comprennent deux joues (62) latérales disposées de part et d'autre des blocs, mobiles en rotation autour d'un axe (37) central entre deux positions, à savoir une position où les blocs sont écartés au maximum les uns des autres, et une position où les blocs sont rapprochés les uns des autres, lesdites joues latérales étant munies de lumières (64) en forme d'arc de cercle, disposées symétriquement par rapport audit axe propres à coopérer avec des pions (36, 38) solidaires des parois latérales extérieures des blocs mobiles entre eux.

6. Appareil selon la revendication 5 dépendante de la revendication 2, caractérisé en ce que le guide de lame de coupe comportant un bloc central pour les coupes en chanfrein, l'axe central est solidaire dudit bloc central et les joues latérales sont munies chacune de deux lumières en forme d'arc de cercle, symétriques par rapport audit axe central et propres à coopérer avec des pions respectivement solidaires des parois latérales des blocs inférieur et supérieur.

7. Appareil selon la revendication 5 dépendante de la revendication 3, caractérisé en ce que le guide de lame de coupe comportant deux blocs médians, l'axe central est solidaire d'un ou de deux éléments intercalaires disposés entre les deux blocs médians et les joues latérales sont munies chacune de quatre lumières en forme d'arc de cercle, symétriques deux à deux par rapport audit axe central, à savoir deux lumières extérieures propres à coopérer avec des pions respectivement solidaires des parois latérales des blocs inférieur et supérieur, et deux lumières intérieures propres à coopérer avec des pions respectivement solidaires des parois latérales des blocs médians.

8. Appareil selon l'une quelconque des revendications 5 à 7, caractérisé en ce que le bord supérieur (200) des joues (62) est courbe, cranté, les moyens de réglage comprenant une pièce basculante (202) en forme de cornière, à cheval et mobile en rotation autour d'un axe solidaire de l'arête externe du bloc (30), comportant deux parties d'extrémité latérales (205) dont les bords périphériques respectifs sont munis d'une lèvre périphérique (207) propre à coopérer avec les crans (201), ladite cornière étant mobile entre une position comprimée où les lèvres sont libérées des crans et une position de rappel par ressort où les lèvres sont encliquées dans lesdits crans.

9. Appareil selon l'une quelconque des revendications précédentes, caractérisé en ce que l'embase inférieure de l'élément support comprend au moins une plaque amovible propre à coopérer en appui avec les condyles fémoraux.

10. Appareil selon l'une quelconque des revendications précédentes, caractérisé en ce que lé bloc inférieur est réglable en hauteur par rapport à l'embase inférieure sur une distance inférieure ou égale à une valeur déterminée.

11. Appareil selon l'une quelconque des revendications précédentes, caractérisé en ce que l'élément support comporte deux paires de colonnes de guidage des blocs reliées entre elles par des pièces d'extrémités et situées de part et d'autre de l'appareil, lesdits blocs comprenant des alésages de passages desdites colonnes pour permettre leur coulissement à frottement doux le long desdites colonnes entre les deux dites positions.

12. Appareil selon l'une quelconque des revendications précédentes, caractérisé en ce que les moyens palpeurs de la partie verticale antérieure du fémur sont déplaçables latéralement par rapport à l'élément support.

13. Appareil selon l'une quelconque des revendications précédentes, caractérisé en ce que le dispositif de coupe fémorale comporte des moyens (50, 81) de réglage de l'angle formé entre l'axe perpendiculaire au guide de lames de coupe et la tige intramédullaire (28).

14. Appareil selon la revendication 13, caractérisé en ce que les moyens (81) de réglage de l'angle sont solidaires de la tige intramédullaire fémorale.

15. Appareil selon la revendication 13, caractérisé en ce que les moyens de réglage de l'angle sont solidaire du bloc supérieur.

16. Appareil selon l'une quelconque des revendications précédentes, caractérisé en ce que le guide de lames de coupe comporte un bloc (95) de coupe distale solidaire du bloc de coupe antérieure, muni d'au moins deux fentes (96) de guidage de lames de coupe distale.

17. Appareil selon l'une quelconque des revendications précédentes, caractérisé
en ce qu'il comporte, de plus, un dispositif (98) de coupe tibiale muni d'une pièce (91) de centrage apte à coopérer avec une tige (101) intramédullaire tibiale, ledit dispositif de coupe tibiale comprenant une tige (103) externe au tibia dont une extrémité (115) est solidaire de ladite pièce et comporte un bloc (109) de coupe muni d'une face courbe de contact avec le tibia, et dont l'autre extrémité est agencée pour être fixée temporairement sur une partie du tibia,
et en ce que ladite extrémité de la tige externe au tibia solidaire de la pièce de centrage est fixée à ladite pièce via un élément intermédiaire (102) agencé pour permettre un réglage de la position du bloc de coupe tibiale dans le plan frontal et dans le plan sagittal, par pivotement autour d'un centre coïncidant avec le centre de gravité ou sensiblement avec le centre de gravité dudit guide de coupe tibiale.

18. Appareil selon la revendication 17, caractérisé en ce que l'élément intermédiaire (102) comprend une pièce ajourée (112) en forme de portion de tube cylindrique définissant une lumière (113) dans laquelle peut se déplacer l'extrémité de la tige externe, ladite extrémité étant munie de deux cales (116, 117 ; 120) hémisphériques ou sensiblement hémisphériques situées de part et d'autre de ladite pièce, lesdites cales étant propres à coopérer à frottement et à être maintenues en pression contre les surfaces cylindriques interne (121') et externe (121) de ladite pièce par des moyens de serrage, le centre des cales hémisphériques et l'axe du cylindre passant par le centre ou sensiblement le centre de gravité (126) dudit bloc de coupe.

## Claims

1. Apparatus for resection of the condyles of the knee in order to position a prosthesis, comprising a femoral cutting device (12) comprising:
• a support member (14) provided with a lower base (15, 16) suitable for co-operating with and bearing against the femoral condyles (17),
• a blade guide (21, 24, 30) for femoral cuts known as anterior cut, anterior chamfered cut, posterior chamfered cut, posterior cut and distal cut, said guide being fixed and comprising at least one block adjustable as to height with respect to the support member,
• an intramedullary femoral rod (28, 80) for orienting and centring said device and
• means (56, 57, 78) for probing the anterior cortical portion (58, 79) of the femur, integral with said guide, characterised
in that the cutting blade guide comprises at least three blocks, of which at least two blocks (21, 24, 30) are moveable with respect to said support member, said blocks being adjustable as to height with respect to each other, one of the blocks comprising means for guiding at least one cut from among the anterior, posterior, anterior chamfered and posterior chamfered cuts, and the other blocks comprising means for guiding the remaining cuts from among said anterior, posterior, anterior chamfered and posterior chamfered cuts.

2. Apparatus according to Claim 1, characterised in that the three blocks (21, 24, 30) are moveable with respect to said support member and comprise a lower block (21) for the posterior cut, a central block (24) for the anterior and posterior chamfered cuts, and an upper block (30) for the anterior cut.

3. Apparatus according to Claim 1, characterised in that the cutting blade guide comprises four blocks moveable with respect to said support member, namely a lower block for the posterior cut, two median blocks comprising a first block for the anterior chamfered cut and a second block for the posterior chamfered cut, and an upper block for the anterior cut.

4. Apparatus according to any one of the preceding claims, characterised in that the device comprises means (61) for proportionally adjusting the spacing between blocks.

5. Apparatus according to Claim 4, characterised in that the means (61) for proportionally adjusting the spacing between blocks comprise two lateral cheeks (62) arranged on either side of the blocks, moveable in rotation about a central axis (37) between two positions, namely a position where the blocks are spaced as far as possible from one another, and a position where the blocks are close to one another, said lateral cheeks being provided with slots (64) in the shape of an arc of a circle, arranged symmetrically with respect to said axis and suitable for co-operating with pins (36, 38) integral with the outer side walls of the blocks moveable between themselves.

6. Apparatus according to Claim 5 dependent on Claim 2, characterised in that since the cutting blade guide comprises a central block for the chamfered cuts, the central axis is integral with said central block and the lateral cheeks are each provided with two slots in the shape of an arc of a circle, symmetrical with respect to said central axis and suitable for co-operating with pins integral, respectively, with the side walls of the lower and upper blocks.

7. Apparatus according to Claim 5 dependent on Claim 3, characterised in that since the cutting blade guide comprises two median blocks, the central axis is integral with one or two inserted members arranged between the two median blocks and the lateral cheeks are each provided with four slots in the shape of an arc of a circle, symmetrical in pairs with respect to said central axis, namely two outer slots suitable for co-operating with pins integral, respectively, with the side walls of the lower and upper blocks, and two inner slots suitable for co-operating with pins integral, respectively, with the side walls of the median blocks.

8. Apparatus according to any one of Claims 5 to 7, characterised in that the upper edge (200) of the cheeks (62) is curved, notched, the adjustment means comprising a tilting part (202) in the shape of an angle section, astride and moveable in rotation about an axis integral with the outer ridge of the block (30), comprising two lateral end portions (205), the respective peripheral edges of which are provided with a peripheral lip (207) suitable for co-operating with the notches (201), said angle section being moveable between a compressed position where the lips are released from the notches and a spring-return position where the lips are clicked into said notches.

9. Apparatus according to any one of the preceding claims, characterised in that the lower base of the support member comprises at least one removable plate suitable for co-operating with and bearing against the femoral condyles.

10. Apparatus according to any one of the preceding claims, characterised in that the lower block is adjustable as to height with respect to the lower base over a distance less than or equal to a predetermined value.

11. Apparatus according to any one of the preceding claims, characterised in that the support member comprises two pairs of columns for guiding the blocks, said columns being connected together by end parts and located on either side of the apparatus, said blocks comprising bore holes passing through said columns in order to allow them to slide with gentle friction along said columns between the two said positions.

12. Apparatus according to any one of the preceding claims, characterised in that the means for probing the anterior vertical portion of the femur are displaceable laterally with respect to the support member.

13. Apparatus according to any one of the preceding claims, characterised in that the femoral cutting device comprises means (50, 81) for adjusting the angle formed between the axis perpendicular to the cutting blade guide and the intramedullary rod (28).

14. Apparatus according to Claim 13, characterised in that the means (81) for adjusting the angle are integral with the femoral intramedullary rod.

15. Apparatus according to Claim 13, characterised in that the means for adjusting the angle are integral with the upper block.

16. Apparatus according to any one of the preceding claims, characterised in that the cutting blade guide comprises a distal cutting block (95) integral with the anterior cutting block, provided with at least two slots (96) for guiding distal cutting blades.

17. Apparatus according to any one of the preceding claims, characterised in that it further comprises a tibial cutting device (98) provided with a centring part (91) suitable for co-operating with a tibial intramedullary rod (101), said tibial cutting device comprising a rod (103) external to the tibia, one end of which (115) is integral with said part and comprises a cutting block (109) provided with a curved face for contact with the tibia, and the other end of which is arranged so as to be fixed temporarily to a portion of the tibia,
and in that said end of the rod external to the tibia, integral with the centring part, is fixed to said part via an intermediate member (102) arranged so as to allow an adjustment of the position of the tibial cutting block in the frontal plane and in the sagittal plane, by pivoting about a centre coinciding with the centre of gravity or substantially with the centre of gravity of said tibial cutting guide.

18. Apparatus according to Claim 17, characterised in that the intermediate member (102) comprises an open-work part (112) in the shape of a portion of a cylindrical tube defining a slot (113) within which the end of the outer rod can be displaced, said end being provided with two hemispherical or substantially hemispherical shims (116, 117; 120) located on either side of said part, said shims being suitable for co-operating by friction, and for being held pressed against the inner (121') and outer (121) cylindrical surfaces of said part by clamping means, the centre of the hemispherical shims and the axis of the cylinder passing through the centre or substantially the centre of gravity (126) of said cutting block.

## Patentansprüche

1. Vorrichtung zur Resektion von Kondylen eines Knies zum Einsetzen einer Prothese mit einer Einrichtung (12) für Femoralschnitte, enthaltend:
- ein Stützelement (14) mit einer unteren Fußplatte (15, 16), die sich an den Femoral-Kondylen (17) abstützen kann,
eine Führung (21, 24, 30) für Messer für Femoralschnitte, bezeichnet mit Vorderschnitt, Vorder-Seitenschnitt, Hinter-Seitenschnitt, Hinterschnitt und Distalschnitt, wobei die Führung ortsfest ist und wenigstens einen Block enthält, der in der Höhe relativ zu dem Stützelement einstellbar ist,
- eine intramodulläre, femorale Stange (28, 80) zur Ausrichtung und Zentrierung der Einrichtung und
- mit der Führung aus einem Stück bestehende Abtastmittel (56, 57, 78) für den vorderen kortikalen Teil (58, 79) des Femurs,
**dadurch gekennzeichnet,**
**dass** die Führung der Schneidmesser wenigstens drei Blöcke enthält, von denen wenigstens zwei Blöcke (21, 24, 30) relativ zu dem Stützelement verstellbar sind, dass die Blöcke in der Höhe gegeneinander verstellbar sind, dass einer der Blöcke Führungsmittel für wenigstens einen Schnitt unter den Vorderschnitten, den Hinterschnitten, den Vorder-Seitenschnitten und den Hinter-Seitenschnitten enthält und die anderen Blöcke Führungsmittel für die übrigen Schnitte unter den Vorderschnitten, Hinterschnitten, Vorder-Seitenschnitten und Hinter-Seitenschnitten enthält.

2. Vorrichtung nach Anspruch 1,
**dadurch gekennzeichnet,**
**dass** die drei Blöcke (21, 24, 30) gegenüber dem Stützelement verstellbar sind und einen unteren Block (21) für den dem Hinterschnitt, einen mittleren Block (24) für die Vorder- und Hinter-Seitenschnitte und einen oberen Block (30) für den Vorderschnitt enthalten.

3. Vorrichtung nach Anspruch 1,
**dadurch gekennzeichnet,**
**dass** die Schneidmesser-Führung vier relativ zu dem Stützelement verstellbare Blöcke enthält, nämlich einen unteren Block für den Hinterschnitt, zwei mittlere Blöcke mit einem ersten Block für den Vorder-Seitenschnitt und einem zweiten Block für den Hinter-Seitenschnitt und einen oberen Block für den Vorderschnitt.

4. Vorrichtung nach einem der vorangehenden Ansprüche,
**dadurch gekennzeichnet,**
**dass** die Einrichtung Mittel (61) zur proportionalen Einstellung der Abstände zwischen den Blöcken enthält.

5. Vorrichtung nach Anspruch 4,
**dadurch gekennzeichnet,**
**dass** die Mittel (61) zur proportionalen Einstellung der Abstände zwischen den Blöcken zwei Seitenteile (62) zu beiden Seiten der Blöcke enthalten, die durch Drehung um eine Mittenachse (37) zwischen zwei Lagen verstellbar sind, nämlich einer Lage, bei der die Blöcke maximal voneinander beabstandet sind, und einer Lage, bei der die Blöcke aneinander angenähert sind, dass die Seitenteile mit Langlöchern (64) in der Form eines Kreisbogens versehen sind, die symmetrisch zu der Achse liegen und mit Lagerzapfen (36, 38) zusammenarbeiten können, die mit den äußeren Seitenwänden der gegeneinander verstellbaren Blöcke aus einem Stück bestehen.

6. Vorrichtung nach Anspruch 5, abhängig von Anspruch 2,
**dadurch gekennzeichnet,**
**dass** die Schneidmesser-Führung einen mittleren Block für die Seitenschnitte enthält, dass die Mittenachse mit dem mittleren Block aus einem Stück besteht und jedes Seitenteil mit Langlöchern in der Form eines Kreisbogens versehen ist, die symmetrisch zu der Mittenachse verlaufen und mit den jeweiligen Lagerzapfen zusammenarbeiten können, die mit den Seitenwänden des unteren und des oberen Blocks aus einem Stück bestehen.

7. Vorrichtung nach Anspruch 5, abhängig von Anspruch 3,
**dadurch gekennzeichnet,**
**dass** die Schneidmesser-Führung zwei mittlere Blöcke enthält, dass die Mittenachse mit einer oder zwei eingefügten Elementen aus einem Stück besteht, die zwischen den beiden mittleren Blöcken liegen, und dass jedes Seitenteil mit vier Langlöchern in der Form eines Kreisbogens versehen ist, von denen jeweils zwei zu der Mittenachse symmetrisch sind, nämlich zwei äußere Langlöcher zum Zusammenarbeiten jeweils mit den mit den Seitenwänden des unteren und oberen Blocks aus einem Stück bestehenden Lagerzapfen, und zwei innere Langlöcher, die mit den mit den Seitenwänden der mittleren Blöcke aus einem Stück bestehenden Lagerzapfen zusammenarbeiten können.

8. Vorrichtung nach einem der Ansprüche 5 bis 7,
**dadurch gekennzeichnet,**
**dass** die Oberkante (200) der Seitenteile (62) gebogen, gekerbt ist, dass die Einstellmittel ein schwenkbares Teil (202) mit Winkelprofil enthalten, das darüber liegt und um eine mit der Außenkante des Blocks (30) aus einem Stück bestehende Achse drehbar ist, dass das schwenkbare Teil zwei seitliche Endteile (205) enthält, deren Umfangskanten jeweils mit einer Umfangslippe (207) versehen sind, die mit den Kerben (201) zusammenarbeiten kann, dass das Teil mit Winkelprofil zwischen einer komprimierten Lage, wo die Lippen aus den Kerben freikommen, und einer unter Federspannung stehenden Lage bewegbar ist, wo die Lippen in die Kerben eingerastet sind.

9. Vorrichtung nach einem der vorangehenden Ansprüche,
**dadurch gekennzeichnet,**
**dass** die untere Fußplatte des Stützelements wenigstens eine verstellbare Platte enthält, die sich an den Femoral-Kondylen abstützen kann.

10. Vorrichtung nach einem der vorangehenden Ansprüche,
**dadurch gekennzeichnet,**
**dass** der untere Block in der Höhe gegenüber der unteren Fußplatte über einen Abstand einstellbar ist, der kleiner oder gleich einem vorbestimmten Wert ist.

11. Vorrichtung nach einem der vorangehenden Ansprüche,
**gekennzeichnet,**
**dass** das Stützelement zwei Paare von Säulen zur Führung der Blöcke enthält, die durch Endteile miteinander verbunden sind und zu beiden Seiten der Vorrichtung liegen, dass die Blöcke Bohrungen für den Durchtritt der Säulen enthalten, um ihre Gleitbewegung mit geringer Reibung entlang den Säulen zwischen den beiden genannten Lagen ermöglichen.

12. Vorrichtung nach einem der vorangehenden Ansprüche,
**dadurch gekennzeichnet,**
**dass** die Abtastmittel des vorderen vertikalen Teils des Femurs seitlich zu dem Stützelement verschiebbar sind.

13. Vorrichtung nach einem der vorangehenden Ansprüche,
**dadurch gekennzeichnet,**
**dass** die femorale Schneidvorrichtung Mittel (50, 81) zur Einstellung des Winkels zwischen der zu der Schneidmesser-Führung senkrechten Achse und der intramedullären Stange (28) enthält.

14. Vorrichtung nach Anspruch 13,
**dadurch gekennzeichnet,**
**dass** die Mittel (81) zur Einstellung des Winkels mit der intramedullären, femoralen Stange aus einem Stück bestehen.

15. Vorrichtung nach Anspruch 13,
**dadurch gekennzeichnet,**
**dass** die Mittel zur Einstellung des Winkels mit dem oberen Block aus einem Stück bestehen.

16. Vorrichtung nach einem der vorangehenden Ansprüche,
**dadurch gekennzeichnet,**
**dass** die Schneidmesser-Führung einen mit dem Block für das vordere Schneiden aus einem Stück bestehenden Block (95) für das distale Schneiden enthält, der mit wenigstens zwei Führungsschlitzen (96) für die Messer für das distale Schneiden versehen ist.

17. Vorrichtung nach einem der vorangehenden Ansprüche,
**dadurch gekennzeichnet,**
**dass** sie zusätzlich eine Einrichtung (98) zum Tibialis-Schneiden enthält, die mit einem Zentrierteil (91) versehen ist, das mit einer intramedullären Tibialis-Stange (101) zusammenarbeiten kann, dass die Einrichtung zum Tibialis-Schneiden extern zu der Tibia eine Stange (103) enthält, deren eines Ende (115) mit dem genannten Teil aus einem Stück besteht und einen Schneidblock (109) enthält, der mit einer gekrümmten Fläche für eine Berührung mit der Tibia versehen ist, und deren anderes Ende dafür ausgebildet ist, vorübergehend auf einem Teil der Tibia befestigt zu werden, und
**dass** das genannte Ende der Stange extern zu der Tibia, die mit dem Zentrierteil aus einem Stück besteht, über ein Zwischenelement (102) an dem Teil befestigt ist, um eine Einstellung der Lage des Tibialis-Schneidblocks in der Stirnebene in der Sagittalebene durch eine Schwenkbewegung um eine Mitte zu ermöglichen, die mit dem Schwerpunkt oder im Wesentlichen mit dem Schwerpunkt der Führung für den Tibialis-Schnitt zusammenfällt.

18. Vorrichtung nach Anspruch 17,
**dadurch gekennzeichnet,**
**dass** das Zwischenelement (102) ein gelochtes Teil (112) in Form eines Teils einer zylindrischen Röhre enthält, das ein Langloch (113) bildet, in das das Ende der äußeren Stange eingreifen kann, dass das Ende mit zwei halbkugelförmigen oder im Wesentlichen halbkugelförmigen Klötzen (116, 117; 120) versehen ist, die zu beiden Seiten des genannten Teils liegen, dass die Klötze mit Reibung zusammenarbeiten können und unter Druck an der inneren (121) und der äußeren (121) zylindrischen Innenfläche des Teils durch Befestigungsmittel gehalten werden, dass der Mittelpunkt der halbkugelförmigen Klötze und die Zylinderachse durch den Schwerpunkt oder im Wesentlichen durch den Schwerpunkt (126) des Schneidblocks verlaufen.
